# EUROPEAN PATENT APPLICATION

(11) **EP 4 360 734 A1**
(43) Date of publication of application: **01.05.2024**
(21) Application number: 21947244.6
(22) Date of filing: 24.06.2021
(51) Int. Cl.: B01D 47/06, B01D 53/78, B01D 53/79, A61L 9/14, B01F 23/20, B01D 53/30

(54) **AIR PURIFICATION APPARATUS**

(30) Priority: 23.06.2021 KR 20210081622
(71) Applicant: CYAG Co., Ltd., Seongnam-si, Gyeonggi-do 13511 (KR)
(72) Inventor: HWANG, Changbae, Seoul 05670 (KR)
(74) Representative: AWA Sweden AB
(86) International application number: PCT/KR2021/007934
(87) International publication number: WO 2022/270653

(57) **Abstract**

One aspect of the present invention provides an air purification apparatus including a purification main body having an air purification space provided therein, the purification main body including an air inlet provided at one side thereof, and an air outlet provided at the other side thereof, a purification fluid spray unit configured to spray a purification fluid into the air purification space so that polluted air introduced into the purification main body is purified in the air purification space, a nano-micro bubble supply unit configured to generate nano-micro bubbles in the purification fluid and supply the purification fluid containing the nano-micro bubbles to the purification fluid spray unit to improve purification power of the purification fluid, and a hydroxyl radical generation unit configured to generate hydroxyl radicals from the purified fluid to purify the polluted air introduced into the purification main body.

## Description

### [Technical Field]

The present invention relates to an air purification apparatus.

### [Background Art]

Exhaust gases emitted by rapid industrialization are destroying the environment. The environmental destruction causes inconvenience everywhere in modern society, and various efforts are being made to cope with the environmental destruction.

Nano-micro bubbles refer to fine bubbles having sizes of several hundreds of nanometers to 10 µm. Unlike typical and general bubbles, i.e., milli-bubbles rise rapidly in water and burst at the water surface, nano-bubbles receive less buoyancy because of small volumes thereof, rise very slowly to the water surface, and thus are maintained in a bubble state in the water over a long period of time. In particular, the nano-bubbles have properties such as a gas dissolution effect, a magnetic pressure effect, and an electrification effect and thus are highly likely to be applied to various fields such as sewage treatment facilities, advanced water purification facilities, soil purification facilities, fishery and agricultural fields, and drainage treatment fields, and cleaning fields.

Hydroxyl radicals refer to materials generated when moisture, oxygen, nitrogen, ozone, and the like in the atmosphere react with ultraviolet rays. The hydroxyl radicals are reactive materials with strong oxidizing power and have high sterilization intensity, strong deodorizing and oxidizing power, and the like.

In addition, the hydroxyl radicals are proven to be harmless to human bodies and effectively kill germs, viruses, mold, and the like.

### [Document of Related Art]

[Patent Document]
(Patent Document 1) Korean Patent No. 10-0903420 (registered on June 10, 2009)

### [Disclosure]

### [Technical Problem]

An object of the present invention provides an air purification apparatus that purifies air by using nano-micro bubbles and hydroxyl radicals.

### [Technical Solution]

One aspect of the present invention provides an air purification apparatus including a purification main body having an air purification space provided therein, the purification main body including an air inlet provided at one side thereof, and an air outlet provided at the other side thereof, a purification fluid spray unit configured to spray a purification fluid into the air purification space so that polluted air introduced into the purification main body is purified in the air purification space, a nano-micro bubble supply unit configured to generate nano-micro bubbles in the purification fluid and supply the purification fluid containing the nano-micro bubbles to the purification fluid spray unit to improve purification power of the purification fluid, and a hydroxyl radical generation unit configured to generate hydroxyl radicals to purify the polluted air introduced into the purification main body.

The nano-micro bubble supply unit may include: a purification fluid container configured to accommodate the purification fluid; a nano-micro bubble generator configured to generate the nano-micro bubbles; and an injection port formed in the purification fluid container to inject a promoter into the purification fluid.

The hydroxyl radical generation unit may include: a hydroxyl radical generation main body connected to at least any one of the air outlet and the purification fluid spray unit; and a flow passageway provided in the hydroxyl radical generation main body so that the purification fluid is introduced into or discharged from the flow passageway.

The hydroxyl radical generation unit may further include a coating layer formed on an inner surface of the flow passageway and configured to generate the hydroxyl radicals by means of a chemical reaction with the purification fluid.

The flow passageway may have a mesh structure so that a plurality of flow passageways is formed, and the nano-micro bubbles may be generated in the flow passageway as a collision, friction, and shear stress occur between particles in the purification fluid while the purification fluid flows.

The purification fluid spray unit may include: a purification fluid inlet port connected to the nano-micro bubble supply unit; a purification fluid tube configured to accommodate the purification fluid; and a purification fluid spray port configured to spray the purification fluid into the purification space.

The purification main body may further include a partition wall disposed to allow the polluted air to meander in the purification space.

The air purification apparatus may further include: an air pollution degree measurement unit provided in the outlet port and configured to measure a pollution degree of the purified air; and an operation control unit connected to the air pollution degree measurement unit and configured to control an operation of the nano-micro bubble supply unit, in which the operation control unit starts the operation of the nano-micro bubble supply unit when the pollution degree measured by the air pollution degree measurement unit exceeds a preset reference value.

The air purification apparatus may further include: an air blower connected to the purification main body so that the polluted air is introduced into the air inlet, and the purified air is discharged to the air outlet.

The purification main body may have a tubular structure having one side connected to the air inlet, and the other side connected to the air outlet, such that the polluted air is introduced into the air inlet, purified while passing through the purification space, and then discharged to the air outlet, the purification fluid spray unit may be disposed in the purification space and spray the purification fluid to the air introduced into the purification main body through the air inlet, and the hydroxyl radical generation unit may be disposed at a rear end of the purification fluid spray unit and generate the hydroxyl radicals in the air to which the purification fluid is sprayed by the purification fluid spray unit.

### [Advantageous Effects]

According to the present invention, it is possible to purify air by using nano-micro bubbles and hydroxyl radicals.

### [Description of Drawings]

FIG. 1 is a view illustrating an air purification apparatus according to an embodiment of the present invention.
FIG. 2A is a transverse cross-sectional view illustrating a hydroxyl radical generation unit of the air purification apparatus according to the embodiment of the present invention.
FIG. 2B is a longitudinal sectional view illustrating the hydroxyl radical generation unit of the air purification apparatus according to the embodiment of the present invention.
FIG. 3 is a view illustrating a modified example of the air purification apparatus according to the embodiment of the present invention.
FIG. 4 is a view illustrating modified examples of the air purification apparatus according to the embodiment of the present invention.
FIG. 5 is a view illustrating an air purification apparatus according to another embodiment of the present invention.

### [Description of Reference Numerals]

- 10:: Purification fluid
- 20:: Polluted air
- 30:: Nano-micro bubble
- 40:: Hydroxyl radical
- 50:: Promoter
- 60:: Energy
- 70:: Purified air
- 100:: Air purification apparatus
- 110:: Purification main body
- 111:: Purification space
- 112:: Air inlet
- 113:: Air outlet
- 114:: Purification fluid discharge port
- 115:: Purification fluid circulation pump
- 120:: Purification fluid spray unit
- 121:: Purification fluid inlet port
- 122:: Purification fluid tube
- 123:: Purification fluid spray port
- 130:: Nano-micro bubble supply unit
- 131:: Purification fluid container
- 132:: Nano-micro bubble generator
- 133:: Injection port
- 140:: Hydroxyl radical generation unit
- 141:: Hydroxyl radical generation main body
- 142:: Flow passageway
- 143:: Coating layer
- 150:: Partition wall
- 160:: Filling layer
- 170:: Structure
- 180:: Dust filter
- 190:: Air pollution degree measurement unit
- 191:: Operation control unit
- 192:: Monitoring unit
- 200:: Air blower
- 210:: Energy supply unit

### [Mode for Disclosure]

The present invention may be variously modified and may have various exemplary embodiments, and particular exemplary embodiments illustrated in the drawings will be described in detail below. However, the description of the exemplary embodiments is not intended to limit the present invention to the particular exemplary embodiments, but it should be understood that the present invention is to cover all modifications, equivalents and alternatives falling within the spirit and technical scope of the present invention. In the description of the present invention, the specific descriptions of publicly known related technologies will be omitted when it is determined that the specific descriptions may obscure the subject matter of the present invention.

The terms such as "first" and "second" may be used to describe various constituent elements, but the constituent elements should not be limited by the terms. These terms are used only to distinguish one constituent element from another constituent element.

The terminology used herein is used for the purpose of describing particular embodiments only and is not intended to limit the present invention. Singular expressions include plural expressions unless clearly described as different meanings in the context. The terms "comprises," "comprising," "includes," "including," "containing," "has," "having" or other variations thereof are inclusive and therefore specify the presence of stated features, integers, steps, operations, elements, components, and/or combinations thereof, but do not preclude the presence or addition of one or more other features, integers, steps, operations, elements, components, and/or combinations thereof.

Hereinafter, embodiments of an air purification apparatus according to the present invention will be described in detail with reference to the accompanying drawings. In the description of the exemplary embodiments with reference to the accompanying drawings, the same or corresponding constituent elements are assigned with the same reference numerals, and the repetitive description thereof will be omitted.

In addition, the terms such as "first" and "second" used hereinafter are merely identification symbols for distinguishing between identical or corresponding constituent elements, and the identical or corresponding constituent elements are not limited by the terms such as "first" and "second".

In addition, in the contact relationship between respective constituent elements, the term "coupling" not only means physical and direct contact between the constituent elements but also be used as a concept including a case in which another constituent element is interposed between the respective constituent elements and in contact with the respective constituent elements.

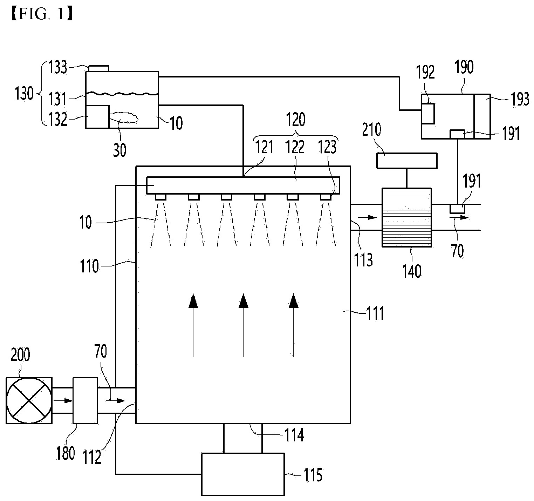

As illustrated in FIGS. 1 to 4, the present embodiment provides an air purification apparatus 100 is an apparatus for purifying polluted air 20 and includes a purification main body 110, a purification space 111, an air inlet 112, an air outlet 113, a purification fluid discharge port 114, a purification fluid circulation pump 115, a purification fluid spray unit 120, a purification fluid inlet port 121, a purification fluid tube 122, purification fluid spray ports 123, a nano-micro bubble supply unit 130, a purification fluid container 131, a nano-micro bubble generator 132, an injection port 133, a hydroxyl radical generation unit 140, a hydroxyl radical generation main body 141, flow passageways 142, coating layers 143, an air blower 200, a dust filter 180, an air pollution degree measurement unit 190, an operation control unit 191, a monitoring unit 192, an energy supply unit 210, and a partition wall 150.

According to the present invention described above, the nano-micro bubble supply unit 130 may supply nano-micro bubbles 30 to the purification fluid 10. When the nano-micro bubbles 30 receive physical impact from the outside or burst autonomously, various types of energy 60 including heat are generated around the nano-micro bubbles 30. The energy 60 may destroy microorganisms such as bacteria or viruses, thereby providing a sterilization effect.

In addition, the nano-micro bubbles 30 may improve the solubility of gases. The nano-micro bubbles 30 may improve the solubility of gases including ozone and oxygen in the purification fluid 10. Hydroxyl radicals 40 are generated in the purification fluid 10 by dissolved ozone and oxygen.

The hydroxyl radicals 40 are natural materials that are harmless to human bodies and exhibit strong oxidizing power capable of chemically decomposing and removing pollutants. The hydroxyl radicals 40 may be generated as ozone and water molecules react, or the hydroxyl radicals 40 may be generated as water molecules receive the energy 60 from the outside.

That is, the hydroxyl radicals 40 may be generated as water molecules are decomposed by the energy 60 generated when the nano-micro bubbles 30 burst in the purification fluid 10. Alternatively, the hydroxyl radicals 40 may be generated as a large amount of dissolved ozone reacts with water molecules by virtue of the improved gas solubility of the purification fluid 10. The hydroxyl radicals 40, which are generated as described above, may improve air purification ability of the purification fluid 10.

In the hydroxyl radical generation unit 140, the hydroxyl radicals 40 may be generated when the hydroxyl radical generation unit 140 receives moisture in the atmosphere or various types of energy 60. In addition, in the hydroxyl radical generation unit 140, the hydroxyl radicals 40 may be generated by a high temperature, visible rays, and the like.

In addition, in the hydroxyl radical generation unit 140, the hydroxyl radicals 40 may be generated by the nano-micro bubbles 30. That is, the nano-micro bubbles 30 may burst while the purification fluid 10, to which the nano-micro bubbles 30 are supplied, passes through the hydroxyl radical generation unit 140, and the hydroxyl radicals 40 may be generated in the hydroxyl radical generation unit 140 by the energy 60 generated in this case. Therefore, the amount of generation of the hydroxyl radicals 40 may increase as the nano-micro bubbles 30 are supplied to the hydroxyl radical generation unit 140.

In addition, the components of the present embodiment may be interchangeably used for a wet scrubber or the like installed in the related art, such that the air purification apparatus 100 may be installed without requiring facilities and costs.

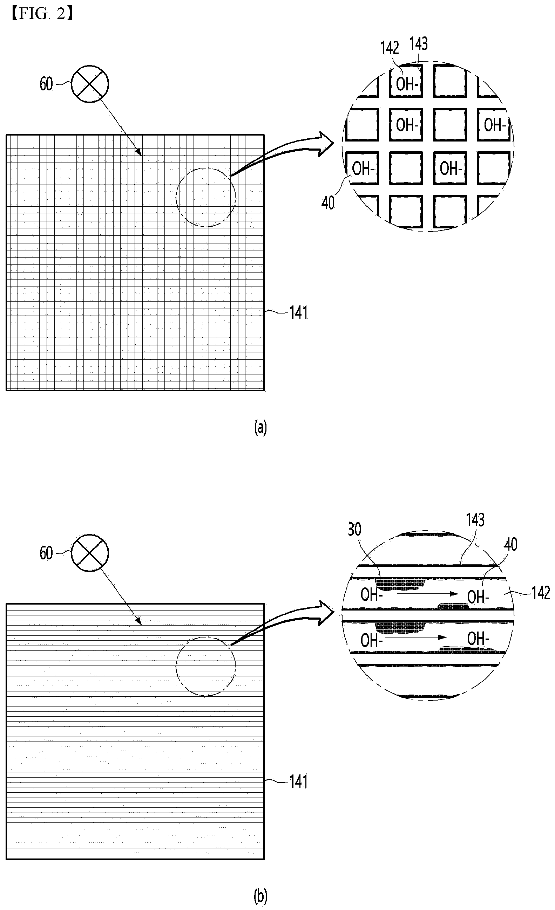

Hereinafter, the components of the air purification apparatus 100 according to the present embodiment will be described more specifically with reference to FIGS. 1 and 2.

As illustrated in FIG. 1, the purification main body 110 may provide the purification space 111 in which air is purified, and the purification main body 110 may have the air inlet 112 provided at one side thereof and configured to allow air to be introduced thereinto, and the air outlet 113 provided at the other side thereof.

As illustrated in FIG. 1, the air inlet 112 may be formed at a lower end of the purification main body 110. As illustrated in FIG. 1, the air outlet 113 may be formed at an upper end of the purification main body 110. Because the introduced warm air tends to rise, the air may flow in the direction from the air inlet 112 formed at the lower end to the air outlet 113 formed at the upper end.

As illustrated in FIG. 1, the purification fluid spray unit 120 may spray the purification fluid 10 into the purification space 111 of the purification main body 110 so that the polluted air 20 is purified in the purification space 111. For example, the purification fluid 10 may be water.

The purification fluid spray unit 120 may include the purification fluid inlet port 121 connected to the nano-micro bubble supply unit 130, the purification fluid tube 122 configured to accommodate the purification fluid 10, and the purification fluid spray ports 123 configured to spray the purification fluid 10 into the purification space 111.

As illustrated in FIG. 1, the purification fluid tube 122 may be disposed above the purification space 111 and spray the purification fluid 10 into the purification space 111 in a gravitational direction. When the purification fluid 10 is sprayed in the gravitational direction, the purification fluid 10 and the polluted air 20 are mixed, and the air may be purified.

As illustrated in FIG. 1, the purification fluid spray ports 123 may spray the purification fluid 10 into the purification space 111. The purification fluid spray port 123 may be provided in the form of a hole through which the purification fluid 10 is dropped by a gravitational force. Alternatively, the purification fluid spray port 123 may be a spray device that atomizes and sprays the purification fluid 10.

The purification fluid spray ports 123 may be variously provided in the form of nozzles including nozzles having small cross-sectional areas and nozzles having large cross-sectional areas. The spray method may spray the purification fluid in various shapes such as a radial shape and a conical shape.

In addition, a purification fluid pressurization device may be provided to pressurize and spray the purification fluid 10. The pressurized purification fluid 10 may be strongly sprayed in a larger range through the purification fluid spray ports 123. As described above, the hydraulic pressure for spraying the purification fluid 10 may be variously set.

As illustrated in FIG. 1, the nano-micro bubble supply unit 130 may include the purification fluid container 131 configured to accommodate the purification fluid 10, the nano-micro bubble generator 132 configured to generate the nano-micro bubbles 30, and the injection port 133 formed in the purification fluid container 131 to inject a promoter 50 into the purification fluid 10.

As illustrated in FIG. 1, the nano-micro bubble supply unit 130 may provide the purification fluid spray unit 120 with the purification fluid 10 containing the nano-micro bubbles 30 generated in the purification fluid 10 by the nano-micro bubble generator 132 to improve air purification power of the purification fluid 10.

The hydroxyl radicals 40 may be generated as the nano-micro bubbles 30 burst in the purification fluid 10 to which the nano-micro bubbles 30 are supplied. The hydroxyl radicals 40 are materials generated as oxygen and ozone react with various types of energy 60, and the hydroxyl radicals 40 are harmless to human bodies and have strong oxidizing power. That is, the hydroxyl radicals 40 oxidize and decompose pollutants, thereby improving the air purification power of the present invention.

In addition, the nano-micro bubbles 30 may increase the gas solubility of the purification fluid 10, thereby increasing the amount of gases such as ozone and the polluted air 20 dissolved in the purification fluid 10. Therefore, the amount of generation of the hydroxyl radicals 40 may be increased by using the dissolved ozone, and a larger amount of polluted air 20 may be dissolved in the purification fluid 10, thereby further improve the purification power of the purification fluid 10.

In addition, various types of energy 60 including instantaneous thermal energy are emitted as the nano-micro bubbles 30 burst. The hydroxyl radicals 40 may be generated as the thermal energy and the like decompose water molecules in the fluid. The energy 60 may generate the hydroxyl radicals 40 in the hydroxyl radical generation unit 140.

As illustrated in FIG. 1, the promoter 50 may be injected into the purification fluid container 131 through the injection port 133. The promoter 50 may include at least one of hydrogen peroxide, titanium dioxide, ozone, and oxygen and serve as an initiator that generates the hydroxyl radicals 40, such that the amount of generation of the hydroxyl radicals 40 in the purification fluid 10 and the hydroxyl radical generation unit 140 may increase.

As illustrated in FIG. 1, the hydroxyl radical generation unit 140 is equipped with the air outlet 113, and the hydroxyl radicals 40 are generated by the purification fluid 10, ozone, oxygen, and the like. The generated hydroxyl radicals 40 may secondarily purify the air 70 purified in the purification main body 110.

In addition, the hydroxyl radical generation unit 140 may be provided in the air inlet 112. The hydroxyl radicals 40 are generated by moisture, ozone, and the like contained in the air introduced into the air inlet 112, and the hydroxyl radicals 40 may purify the polluted air 20.

A moisture content of the purified air 70, which reaches the hydroxyl radical generation unit 140, may be increased by the purification fluid 10 in the purification space 111. In addition, a part of the purification fluid 10 may be mixed with the purified air 70 and reach the hydroxyl radical generation unit 140.

As described above, when the moisture content of the purified air 70 increases or the purification fluid 10 reaches the hydroxyl radical generation unit 140, physical and/or chemical energy (e.g., heat, vibration, pressure, photosynthesis, etc.) may be applied to the hydroxyl radical generation unit 140, such that the hydroxyl radicals 40 may be generated by means of a chemical reaction between the moisture and the purification fluid.

As illustrated in FIG. 2, the hydroxyl radical generation unit 140 may include the hydroxyl radical generation main body 141 into or from which the fluid may be introduced or discharged, and the flow passageways 142 in which the hydroxyl radicals 40 are generated.

As illustrated in FIG. 2, the nano-micro bubbles 30 may be generated again by various structures in the flow passageways 142, and the hydroxyl radicals 40 may be generated from the coating layers 143 formed in the flow passageways 142.

As illustrated in FIG. 2A, the flow passageways 142 may have a mesh structure. The mesh structure may be formed as members of the flow passageways 142 intersect one another so that the plurality of flow passageways 142 is formed.

When the plurality of flow passageways 142 having the mesh structure is formed as described above, a contact area between the purification fluid 10 and the flow passageways 142 may increase, such that a contact area between the purification fluid 10 and the coating layers 143 may increase, thereby increasing the amount of generation of the hydroxyl radicals 40. The flow passageways 142 may be formed as irregular flow paths by the mesh structure.

In addition, as illustrated in FIG. 2B, the purification fluid 10 flows in the flow passageways 142, and collisions, friction, and cavitation occur between particles in the purification fluid 10, such that the nano-micro bubbles 30 may be generated again. The purification fluid 10 may be mixed with the purified air 70 in the purification space 111 and reach the hydroxyl radical generation unit 140.

The above-mentioned collision and frictional operation may allow the gas to be dissolved in the fluid more properly and further atomize the fluid so that the nano-micro bubbles 30 having sizes of at least several nanometers (nm) to several tens of micrometers (µm) may be generated again.

The flow passageway 142 may have a small cross-section and a long length so that the above-mentioned collision and frictional operation occur properly. The flow passageway 142 may be formed to be much smaller in cross-section than the air outlet 113, and the length of the passageway is set to be as large as several ten times to several hundred times the width of passageway, such that the flow passageway 142 may be formed to be long and narrow.

That is, the hydroxyl radicals 40 may be generated at front ends of the flow passageways 142 as the nano-micro bubbles 20, which are supplied to the purification fluid 10, burst, and the nano-micro bubbles 30 may be generated again at rear ends of the flow passageways 142. Therefore, the hydroxyl radicals 40 may be generated as the nano-micro bubbles 30, which are generated again at the rear ends of the flow passageways 142, burst, and the nano-micro bubbles 30, together with the purified air 70, may be discharged into the atmosphere. As described above, the discharged nano-micro bubbles 30 may purify outside air.

When the nano-micro bubbles 30 are generated again in the flow passageways 142 as described above, the amount of generation of the hydroxyl radicals 40 using the nano-micro bubbles 30 in the flow passageways 142 may increase, such that the air purification ability may be improved, and the outside air may be purified.

As illustrated in FIG. 2, the coating layers 143 may be formed in the flow passageways 142 and generate the hydroxyl radicals 40 capable of purifying the polluted air 20. The coating layer 143 may be made of a material including at least any one of titanium oxide, phosphoric acid, and tourmaline.

As illustrated in FIG. 2, the hydroxyl radicals 40 may be generated from the coating layers 143 by various types of energy 60. The energy 60 used to generate the hydroxyl radicals 40 may include at least any one of thermal energy, light energy, and vibrational energy.

In addition, the energy 60 may be the energy 60 generated as the nano-micro bubbles 30 burst. The energy 60, which is generated as the nano-micro bubbles 30 burst, may include a high temperature (4,000 to 6,000 degrees Celsius), ultrahigh-frequency waves (5 MHz), and the like that are generated instantaneously.

Therefore, the amount of generation of the hydroxyl radicals 40 in the hydroxyl radical generation unit 140 may be increased by the nano-micro bubbles 30.

As illustrated in FIG. 1, the operation control unit 191 is connected to the air pollution degree measurement unit 190 provided in the air outlet 113 and configured to measure a pollution degree of the purified air 70, and the operation control unit 191 may control an operation of the nano-micro bubble supply unit 130.

As illustrated in FIG. 1, the air pollution degree measurement unit 190 may be disposed at a rear end of the hydroxyl radical generation unit 140 based on a flow direction of the fluid. Therefore, the air pollution degree measurement unit 190 may measure the air pollution degree of the air that has been subjected to the purification process in the hydroxyl radical generation unit 140.

In case that the pollution degree measured by the air pollution degree measurement unit 190 exceeds a reference value preset in the operation control unit 191, the operation control unit 191 may start the operation of the nano-micro bubble supply unit 130.

As described above, the operation control unit 191 may control the operation of the nano-micro bubble supply unit 130. In case that a purification degree of the purified air 70 in the air outlet 113 is low, the operation control unit 191 may automatically start the operation of the nano-micro bubble generator 132.

That is, the content of the nano-micro bubbles 30 in the purification fluid 10 may be automatically increased, and the air purification degree in the purification space 111, the hydroxyl radical generation unit 140, and the like may be improved by using the above-mentioned air purification ability of the nano-micro bubbles 30.

As illustrated in FIG. 1, the monitoring unit 192 may monitor the air pollution degree measurement unit 190 and the operation control unit 191. The monitoring unit 192 may identify the pollution degree of the air and identify whether various air purification apparatuses 100 including the nano-micro bubble supply unit 130 operate normally.

The monitoring unit 192 may be connected to a mobile device of a manager of the air purification apparatus 100, such that the manager may remotely identify the pollution degree.

As illustrated in FIG. 1, the air blower 200 may be connected to the purification main body 110 and introduce the polluted air 20 into the air inlet 112. The air blower 200 may generate a flow of the introduced air in the direction from the air inlet 112 to the air outlet 113 and discharge the polluted air 20 to the air outlet 113 through the purification space 111 of the purification main body 110.

As illustrated in FIG. 1, the purification fluid discharge port 114 may be disposed in a lower-end surface of the purification main body 110 to discharge the purification fluid 10, which is sprayed from the purification fluid spray unit 120, to the outside of the purification main body 110.

As illustrated in FIG. 1, the purification fluid circulation pump 115 may be connected to the purification fluid discharge port 114 to reuse the purification fluid 10 discharged to the purification fluid discharge port 114. The purification fluid circulation pump 115 may be connected to the nano-micro bubble generation unit, such that the purification fluid 10 may be supplied with the nano-micro bubbles 30 and reused. In addition, the purification fluid circulation pump 115 may be connected to the purification fluid spray unit 120, such that the purification fluid 10 may be immediately reused.

As illustrated in FIG. 1, the energy supply unit 210 may provide the energy 60 to the hydroxyl radical generation unit 140 to promote the generation of the hydroxyl radicals 40. The energy supply unit 210 may be installed as a mechanism, such as a lighting device, a pressing device, or a vibration generator, that supplies various types of energy 60.

As illustrated in FIG. 1, the dust filter 180 may be disposed in the air inlet 112 to purify the polluted air 20. The dust filter 180 is installed and primarily filters out dust having a relatively large particle size, such that the purification degree of the polluted air 20 may be improved.

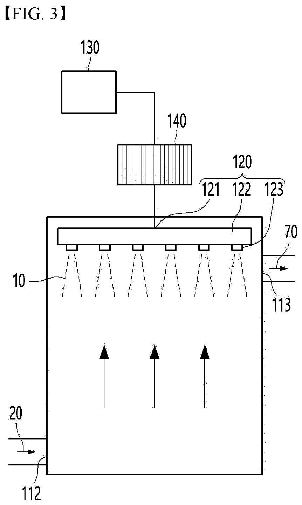

Next, a modified example of the air purification apparatus 100 according to the embodiment will be described with reference to FIG. 3.

As illustrated in FIG. 3, the hydroxyl radical generation unit 140 may be connected to the purification fluid spray unit 120.

When the hydroxyl radical generation unit 140 is connected to the purification fluid spray unit 120 as described above, a large amount of purification fluid 10, which is supplied from the nano-micro bubble supply unit 130 to the purification fluid spray unit 120, may pass through the flow passageways 142, unlike a configuration in which the hydroxyl radical generation unit 140 is positioned in the air outlet 113.

As the large amount of purification fluid 10 passes through the flow passageways (142 in FIG. 2), the collision, friction, and cavitation more actively may occur between the particles in the purification fluid 10, such that a larger number of nano-micro bubbles 30 may be generated again.

In addition, the hydroxyl radicals 40 may be generated by the energy 60 generated as the nano-micro bubbles 30 burst at a front end of the hydroxyl radical generation unit 140, and the nano-micro bubbles 30 are generated again in the purification fluid 10 at the rear end of the hydroxyl radical generation unit 140, such that the purification power of the purification fluid 10 may be improved.

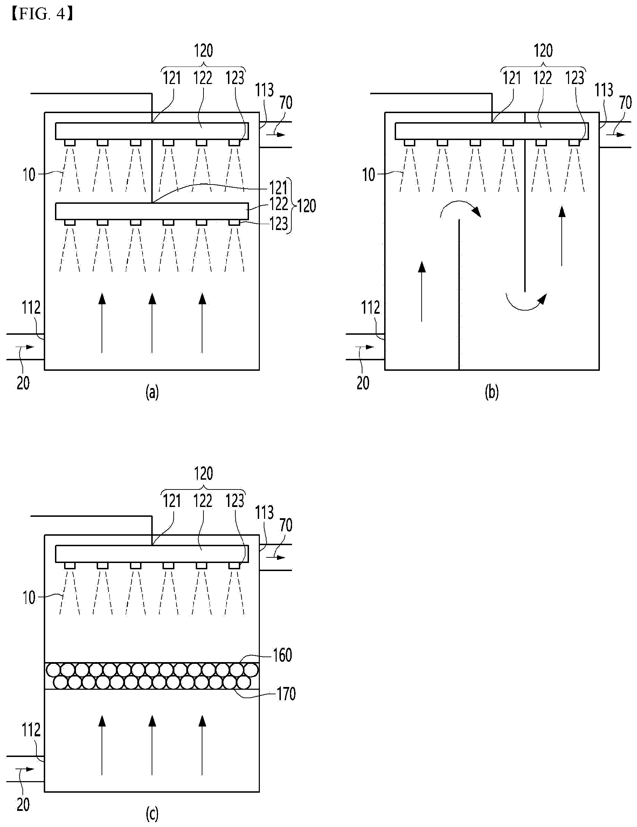

Next, other modified examples of the air purification apparatus 100 according to the embodiment will be described with reference to FIG. 4.

Because the configuration and the operation/effect according to the configuration of the air purification apparatus 100 according to the present embodiment including the purification main body 110, the purification space 111, the air inlet, the air outlet, the air blower 200, the purification fluid discharge port 114, the purification fluid circulation pump 115, the purification fluid spray unit 120, the purification fluid inlet port 121, the purification fluid tube 122, the purification fluid spray port 123, the nano-micro bubble supply unit 130, the nano-micro bubble supply unit 130, the purification fluid container 131, the nano-micro bubble generator 132, the injection port 133, the hydroxyl radical generation unit 140, the hydroxyl radical generation main body 141, the flow passageway 142, the coating layer 143, the dust filter 180, the air pollution degree measurement unit 190, the operation control unit 191, the monitoring unit 192, and the energy supply unit 210 have been already described with reference to the above-mentioned embodiments, specific descriptions thereof will be omitted, and the description will focus on various embodiments of the purification main body 110 that are different from the above-mentioned embodiment.

As illustrated in FIG. 4A, the purification fluid spray unit 120 may be provided as a plurality of purification fluid spray units 120 disposed in the purification space 111. In case that the plurality of purification fluid spray units 120 is disposed, a large amount of purification fluid 10 may be sprayed at one time, such that the amount of purification fluid 10 capable of dissolving the polluted air 20 may increase, and as a result, a large amount of polluted air 20 may be purified at one time.

As illustrated in FIG. 4B, the partition wall 150 may be disposed so that the polluted air 20 flows while meandering in the purification space 111. The partition wall 150 may be installed in an upward/downward direction so that the purification fluid 10 reaches a lower surface of the purification space 111 when the purification fluid spray unit 120 sprays the purification fluid 10. In addition, the partition wall 150 may be provided as a plurality of partition walls 150 disposed in the purification space 111.

In case that the plurality of partition walls 150 is installed in the upward/downward direction as described above, the polluted air 20 introduced into the purification main body 110 flows linearly in an 'S' shape to the air outlet. Therefore, the time for which the polluted air 20 stays in the purification main body 110 is prolonged, and the time for which the polluted air 20 is in contact with the purification fluid 10 is prolonged, such that the purification power of the polluted air 20 may be improved.

In addition, the partition wall 150 may be configured as a porous plate having a plurality of holes. In order to minimize airflow resistance when the polluted air moves in the purification space, a part of the polluted air may move to the holes of the porous plate, and another part of the polluted air may move while bypassing the holes of the porous plate.

As illustrated in FIG. 4C, a filling layer 160 may be disposed in the purification space 111 of the purification main body 110 and filled with a filling material. The filling layer 160 may be filled with a structure 170 for improving porosity to increase the time for which the purification fluid 10 and the polluted air 20 are in contact with each other.

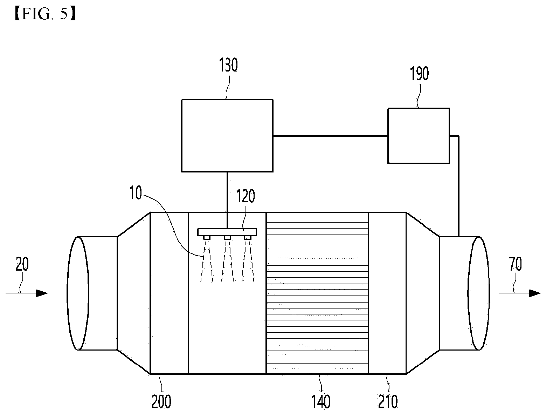

Next, the air purification apparatus 100 according to another embodiment of the present invention will be described with reference to FIG. 5.

The air purification apparatus 100 according to the present embodiment will be described while focusing on the configurations of the purification main body 110, the air inlet 112, the air outlet 113, the purification space 111, the purification fluid spray unit 120, the hydroxyl radical generation unit 140, the air blower 200, the energy supply unit 210, and the operation control unit 191 that are different from those of the above-mentioned embodiment.

As illustrated in FIG. 5, the purification main body 110 has a tubular structure having one side connected to the air inlet 112, and the other side connected to the air outlet 113, such that the polluted air 20 may be introduced through the air inlet 112, purified while passing through the purification space 111, and then discharged to the air outlet 113.

As illustrated in FIG. 5, the purification fluid spray unit 120 may be disposed in the purification space 111 of the purification main body 110 and spray the purification fluid 10 to the air introduced into the purification main body 110 through the air inlet 112.

As illustrated in FIG. 5, the hydroxyl radical generation unit 140 may be disposed at a rear end of the purification fluid spray unit 120 may generate the hydroxyl radicals 40 in the air to which the purification fluid 10 is sprayed by the purification fluid spray unit 120.

As illustrated in FIG. 5, the air blower 200 may be disposed at a front end of the purification main body 110 and allow the air to be introduced into the air inlet 112.

As illustrated in FIG. 5, the energy supply unit 210 may be disposed at the rear end of the hydroxyl radical generation unit 140, such that the amount of generation of the hydroxyl radicals 40 in the hydroxyl radical generation unit 140 may increase.

The air purification apparatus, which has the tubular structure as described above, may be connected to the air purification apparatus in the related art. For example, the air purification apparatus may be installed in a suction part or a discharge part of the wet scrubber and improve air purification power of the wet scrubber. In addition, the air purification apparatus may be connected to a ventilation system in a large-scale building, a ventilation system of an underground shopping center, or the like.

While the exemplary embodiments of the present invention have been described above, those skilled in the art may variously modify and change the present invention by adding, changing, deleting or modifying constituent elements without departing from the spirit of the present invention disclosed in the claims, and the modification and change also belong to the scope of the present invention.

## Claims

1. An air purification apparatus comprising:
a purification main body having an air purification space provided therein, the purification main body including an air inlet provided at one side thereof, and an air outlet provided at the other side thereof;
a purification fluid spray unit configured to spray a purification fluid into the air purification space so that polluted air introduced into the purification main body is purified in the air purification space;
a nano-micro bubble supply unit configured to generate nano-micro bubbles in the purification fluid and supply the purification fluid containing the nano-micro bubbles to the purification fluid spray unit to improve purification power of the purification fluid; and
a hydroxyl radical generation unit configured to generate hydroxyl radicals to purify the polluted air introduced into the purification main body.

2. The air purification apparatus of claim 1, wherein the nano-micro bubble supply unit comprises:
a purification fluid container configured to accommodate the purification fluid;
a nano-micro bubble generator configured to generate the nano-micro bubbles; and
an injection port formed in the purification fluid container to inject a promoter into the purification fluid.

3. The air purification apparatus of claim 1, wherein the hydroxyl radical generation unit comprises:
a hydroxyl radical generation main body connected to at least any one of the air outlet and the purification fluid spray unit; and
a flow passageway provided in the hydroxyl radical generation main body so that the purification fluid is introduced into or discharged from the flow passageway.

4. The air purification apparatus of claim 3, wherein the hydroxyl radical generation unit further comprises a coating layer formed on an inner surface of the flow passageway and configured to generate the hydroxyl radicals by means of a chemical reaction with the purification fluid.

5. The air purification apparatus of claim 3, wherein the flow passageway has a mesh structure so that a plurality of flow passageways is formed, and
wherein the nano-micro bubbles are generated in the flow passageway as a collision, friction, and shear stress occur between particles in the purification fluid while the purification fluid flows.

6. The air purification apparatus of claim 1, wherein the purification fluid spray unit comprises:
a purification fluid inlet port connected to the nano-micro bubble supply unit;
a purification fluid tube configured to accommodate the purification fluid; and
a purification fluid spray port configured to spray the purification fluid into the purification space.

7. The air purification apparatus of claim 1, wherein the purification main body further comprises a partition wall disposed to allow the polluted air to meander in the purification space.

8. The air purification apparatus of claim 1, further comprising:
an air pollution degree measurement unit provided in the outlet port and configured to measure a pollution degree of the purified air; and
an operation control unit connected to the air pollution degree measurement unit and configured to control an operation of the nano-micro bubble supply unit,
wherein the operation control unit starts the operation of the nano-micro bubble supply unit when the pollution degree measured by the air pollution degree measurement unit exceeds a preset reference value.

9. The air purification apparatus of claim 1, further comprising:
an air blower connected to the purification main body so that the polluted air is introduced into the air inlet, and the purified air is discharged to the air outlet.

10. The air purification apparatus of claim 1, wherein the purification main body has a tubular structure having one side connected to the air inlet, and the other side connected to the air outlet, such that the polluted air is introduced into the air inlet, purified while passing through the purification space, and then discharged to the air outlet,
wherein the purification fluid spray unit is disposed in the purification space and sprays the purification fluid to the air introduced into the purification main body through the air inlet, and
wherein the hydroxyl radical generation unit is disposed at a rear end of the purification fluid spray unit and generates the hydroxyl radicals in the air to which the purification fluid is sprayed by the purification fluid spray unit.
